(19) Europäisches Patentamt
European Patent Office
Office européen des brevets

(11)  **EP 2 491 001 B1**

(12)  **EUROPÄISCHE PATENTSCHRIFT**

(45) Veröffentlichungstag und Bekanntmachung des Hinweises auf die Patenterteilung:
**30.04.2014 Patentblatt 2014/18**

(51) Int Cl.:
***C07C 209/36*** *(2006.01)*   ***C07C 265/14*** *(2006.01)*
***C07C 211/46*** *(2006.01)*

(21) Anmeldenummer: **10766063.1**

(22) Anmeldetag: **20.10.2010**

(86) Internationale Anmeldenummer:
**PCT/EP2010/065782**

(87) Internationale Veröffentlichungsnummer:
**WO 2011/048134 (28.04.2011 Gazette 2011/17)**

(54) **VERFAHREN ZUR HERSTELLUNG UND BEVORZUGT DESTILLATIVEN AUFARBEITUNG VON DIPHENYLMETHAN-DIISOCYANAT (MDI)**

PROCESS FOR PREPARATION AND PREFERABLY DISTILLATIVE PROCESSING OF DIPHENYLMETHANE DIISOCYANATE (MDI)

PROCÉDÉ POUR LA PRODUCTION ET LE RETRAITEMENT DE PRÉFÉRENCE PAR DISTILLATION DE DIISOCYANATE DE DIPHÉNYLMÉTHANE (MDI)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.10.2009 EP 09173515**

(43) Veröffentlichungstag der Anmeldung:
**29.08.2012 Patentblatt 2012/35**

(73) Patentinhaber: **BASF SE**
**67056 Ludwigshafen (DE)**

(72) Erfinder:
 • **SCHNEIDER, Christian**
**68165 Mannheim (DE)**
 • **ZAFRED, Nikolaus**
**67059 Ludwigshafen (DE)**
 • **HEUSSLER, Andreas**
**67454 Hassloch (DE)**
 • **DENISSEN, Leo**
**Brasschaat (BE)**
 • **KÖNIGSMANN, Lucia**
**70197 Stuttgart (DE)**

(56) Entgegenhaltungen:
**EP-A1- 0 696 574   EP-B1- 1 053 222**
**WO-A2-01/64333   DE-B- 1 114 820**
**DE-B- 1 133 394**

**Beschreibung**

Die Erfindung betrifft ein Verfahren zur Herstellung und destillativen Aufarbeitung von Diphenylmethan-Diisocyanat, im Folgenden abgekürzt als MDI bezeichnet, ausgehend von einem Benzol enthaltenden Einsatzstrom, wobei Benzol zu Nitrobenzol nitriert, dieses zu Anilin hydriert, Anilin mit Formaldehyd zu Methylendiphenylamin (MDA) umgesetzt und MDA zu MDI phosgeniert wird. Hierbei werden in sämtlichen Verfahrensschritten nicht die reinen Produkte, sondern zunächst jeweils Produktgemische erhalten, die vor der Weiterverarbeitung aufgereinigt werden.

Zur Aufarbeitung der Produktgemische aus den unterschiedlichen Verfahrensstufen ist der Einsatz von Dampf auf zwei unterschiedlichen Druckstufen erforderlich. Aus der exothermen Verfahrensstufe, der katalytischen Hydrierung von Nitrobenzol zu Anilin, wurde bislang Dampf auf einer einzigen Druckstufe erhalten. Für einen Teil der Verfahrensschritte im Gesamtverfahren war es zusätzlich erforderlich, Dampf auf einem höheren Druckniveau von außerhalb der Anlage zuzukaufen.

Es war demgegenüber Aufgabe der Erfindung, ein Verfahren zur Herstellung und destillativen Aufarbeitung von MDI ausgehend von einem Benzol enthaltenden Einsatzstrom zur Verfügung zu stellen, das energetisch autark oder weitgehend autark betrieben werden kann.

Die Lösung besteht in einem Verfahren zur Herstellung und destillativen Aufarbeitung von Diphenylmethan-Diisocyanat (MDI), ausgehend von einem Benzol enthaltenden Einsatzstrom, wobei in

Verfahrensstufe I der Benzol enthaltende Einsatzstrom zu einem Nitrobenzol enthaltenden Produktgemisch nitriert, in

Verfahrensstufe II das Nitrobenzol enthaltende Produktgemisch bevorzugt destillativ aufgearbeitet wird, unter Erhalt eines Nitrobenzol enthaltenden Einsatzstromes, der in

Verfahrensstufe III einer Hydrierung zu einem Anilin enthaltenden Produktgemisch in einem Wirbelschichtreaktor, unter Abführung der Reaktionswärme durch Siedekühlung mit Wasser, wobei Dampf entsteht, zugeführt wird, wobei in

Verfahrensstufe IV das Anilin enthaltende Produktgemisch bevorzugt destillativ aufgearbeitet wird, unter Erhalt eines Anilin enthaltenden Einsatzstromes, der in

Verfahrensstufe V mit Formaldehyd in Gegenwart saurer Katalysatoren zu einem Methylendiphenylamin (MDA) enthaltenden Strom umgesetzt wird, der in

Verfahrensstufe VI bevorzugt destillativ aufgereinigt, und in

Verfahrensstufe VII einer Phosgenierung zu einem MDI enthaltenden Produktgemisch zugeführt wird, das in

Verfahrensstufe VIII bevorzugt destillativ zu Rein-MDI aufgearbeitet wird,

das dadurch gekennzeichnet ist, dass in Verfahrensstufe III Dampf auf zwei unterschiedlichen Druckstufen erzeugt wird, der den Energiebedarf für die Verfahrensstufen IV, VI und VIII teilweise oder vollständig deckt, indem zwei baugleiche Wirbelschichtreaktoren eingesetzt werden, wobei

- ein erster Wirbelschichtreaktor mit einer Anilinlast betrieben wird, für die die Wirbelschichtreaktoren ausgelegt wurde und Dampf auf einer ersten, niedrigeren Druckstufe liefert, und

- ein zweiter Wirbelschichtreaktor mit einer gegenüber dem ersten Wirbelschichtreaktor soweit erniedrigter Last betrieben wird, dass der zweite Wirbelschichtreaktor Dampf auf der für die Verfahrensstufen IV, VI und VIII erforderlichen höheren Druckstufe liefert.

Verfahrensstufe I

Die Verfahrensstufe I, die Nitrierung eines Benzol enthaltenden Einsatzstromes zu einem Nitrobenzol enthaltenden Produktgemisch, wird technisch vorwiegend kontinuierlich, durch Zusammenmischen eines Gemisches aus Salpetersäure und Schwefelsäure, so genannter Mischsäure, mit Benzol durchgeführt. Da die Nitrierung im Wesentlichen in der Säurephase erfolgt, muss das Benzol aus der organischen Phase in die Säurephase diffundieren, wo es mit der dort vorhandenen Salpetersäure zu Nitrobenzol reagiert.

Ein großtechnisches Verfahren zur Nitrierung von Benzol ist in EP-B 0 771 783 beschrieben, wonach eine Mischeinrichtung eingesetzt wird, die als Treibstrahldüse ausgebildet ist, mit einem zentralen Innenrohr, durch das die Mischsäure geleitet wird, die als Treibstrahl für die Mischeinrichtung fungiert und den Benzol enthaltenden Einsatzstrom einsaugt, der in den das Innenrohr umgebenden Ringraum eingeleitet wird.

Dieses Verfahren wurde von BASF SE weiter verbessert: Nach dem BASF-Verfahren wird die Reaktion adiabatisch in einem Rohrreaktor, in einem großen Überschuss von Schwefelsäure durchgeführt, wobei die Schwefelsäure die Reaktion katalysiert und als Wärmeträger fungiert, der die hohe frei werdende Reaktionswärme ($\Delta H_R$ = -117 kJ $\cdot$ mol$^{-1}$) abführt. Die Temperatur steigt von ursprünglich 90°C auf 135°C. Nach der Reaktion wird die organische Phase von der sauren Phase abgetrennt. Die Schwefelsäure wird aufkonzentriert und in das Verfahren recycliert. Das Nitrobenzol enthaltende Produktgemisch, auch als Roh-Nitrobenzol bezeichnet, wird mit Wasser und Lauge in einer Mixer-Settler-Kaskade gewaschen, um die noch vorhandene Schwefelsäure zu neutralisieren und Verunreinigungen zu entfernen.

Die Nitrierung zu Nitrobenzol kann bevorzugt in einem Rohrreaktor mit statischen Mischern durchgeführt werden, wie er in WO 01/64333 beschrieben ist.

Verfahrensstufe II

In Verfahrensstufe II wird das Nitrobenzol enthaltende Produktgemisch bevorzugt destillativ aufgereinigt, wobei Benzol und Wasser als Leichtsieder abgetrennt und ein Nitrobenzol enthaltender Einsatzstrom abgezogen wird, der der Verfahrensstufe III, der katalytischen Hydrierung zu einem Anilin enthaltenden Produktgemisch, zugeführt wird.

Verfahrensstufe III

In Verfahrensstufe III wird die katalytische Hydrierung eines Nitrobenzol enthaltenden Einsatzstromes zu einem Anilin enthaltenden Produktgemisch erfindungsgemäß in einem Wirbelschichtreaktor durchgeführt.

Die großtechnische Durchführung des Verfahrens in Wirbelschichtreaktoren ist schon lange etabliert und insbesondere in der deutschen Patentschrift DE 1 114 820 und dem Zusatzpatent hierzu, der DE 1 133 394, beschrieben. Die katalytische Hydrierung von Nitrobenzol ist bekanntlich stark exotherm, so dass die Einhaltung der Reaktionstemperatur Schwierigkeiten bereitet. Die Einhaltung der Reaktionstemperatur ist insbesondere erforderlich, um zu verhindern, dass Verharzungen auf dem Katalysator auftreten und damit die Aktivität des Katalysators in kurzer Zeit nachlässt. Um diese Probleme zu beherrschen, wird im Verfahren der DE 1 114 820 die Reaktion in einem Wirbelschichtreaktor durchgeführt, wobei der Ausgangsstoff Nitrobenzol flüssig an mehreren Stellen auf verschiedenen Höhen und der für die Hydrierung notwendige Wasserstoff mit dem Nitrobenzol zusammen und/oder am Boden des Wirbelschichtreaktors zugeführt wird. Zur Abführung der Reaktionswärme ist im Wirbelschichtreaktor ein Rohrsystem mit einem darin zirkulierenden Wärmeträger, insbesondere Wasser, vorgesehen. Erfindungsgemäß wird die Reaktionswärme durch Siedekühlung mit Wasser abgeführt, wobei Dampf entsteht. Zusätzlich kann auch eine Mantelkühlung vorgesehen sein.

Im Verfahren nach dem Hauptpatent DE-A 1 114 820 wird drucklos gearbeitet, abweichend hiervon im Verfahren nach dem Zusatzpatent DE-A 1 133 394 bei erhöhtem Druck von mindestens 3 Atmosphären Überdruck, wodurch eine längere Lebensdauer des Katalysators erreicht werden soll.

Als Katalysatoren kommen die Schwermetalle der V. bis VII. Gruppe des Periodensystems sowie der Eisen- und Platingruppe, z.B. Kupfer, Molybdän, Wolfram, Nickel, Kobalt oder Gemische dieser Elemente, sowie ihre Oxyde, Sulfide oder Halogenide, gegebenenfalls zusammen mit Bor oder Borverbindungen, in Betracht. Sie können auch auf Träger, wie Tonerde, natürliche und künstliche Silikate, Bimsstein, Eisenoxyd, Magnesia, Zinkoxyd, Zirkonoxyd, Titanoxyd oder Thoriumoxyd, aufgetragen sein. Die Träger können mit Brom, Jod, Fluor oder Chlor behandelt sein. Der Katalysator wird in körniger Form oder als Pulver angewandt.

Aufgrund der sehr guten Wärmeabfuhreigenschaften des Wirbelbettes, in dem zur Abführung von Reaktionswärme Wärmestromdichten im Bereich von 10 bis 100 kW pro Quadratmeter realisiert werden können, kann der Wirbelbettreaktor für die favorisierte isotherme Reaktionsführung deutlich einfacher im Vergleich zu Rohrreaktoren gestaltet werden, die aufwändig gekühlt werden müssen.

Als nachteilig erweist sich die Wirbelschicht jedoch hinsichtlich des Stoffübergangs, da durch die Bildung von feststoffarmen Gasblasen der Kontakt zwischen Katalysator und Reaktionspartnern in bekannter Weise limitiert ist. Dies hat zur Folge, dass ein Teil der aromatischen Nitroverbindungen nicht in Kontakt mit dem wirbelnden Trägerkatalysator kommt und die Reaktionszone nicht umgesetzt verlässt. Dadurch sinkt nicht nur der Umsatz, sondern es ergeben sich auch weitere Nachteile: Beispielsweise erweist sich nicht umgesetztes Nitrobenzol im Anilin störend bei der Herstellung von Diphenylmethandiisocyanat (MDI), das ein wichtiges Zwischenprodukt in der Polyurethan-Wertschöpfungskette ist.

Die Verfahrensstufe III, die katalytische Hydrierung von Nitrobenzol zu Anilin, wird daher vorteilhaft in einem verbesserten Wirbelschichtreaktor durchgeführt, wie er in WO 2008/034770 vorgeschlagen ist, und zwar in einem Wirbelschichtreaktor, in dem Einbauten vorgesehen sind, die die Wirbelschicht in eine Mehrzahl von horizontal sowie eine Mehrzahl von vertikal im Wirbelschichtreaktor angeordneten Zellen aufteilen, mit Zellwänden, die gasdurchlässig sind und die Öffnungen aufweisen, die eine Austauschzahl des heterogenen, partikelförmigen Katalysators in vertikaler Richtung im Bereich von 1 bis 100 Litern pro Stunde pro Liter Reaktorvolumen gewährleisten.

Besonders bevorzugt werden als Einbauten in der Wirbelschicht Kreuzkanalpackungen eingesetzt, d.h. Packungen mit in vertikaler Richtung im Wirbelbettreaktor parallel zueinander angeordneten geknickten gasdurchlässigen Metallblechen, Streckmetall- oder Gewebelagen, mit Knickkanten, die Knickflächen mit einem von Null verschiedenen Neigungswinkel zur Vertikalen ausbilden, und wobei die Knickflächen aufeinander folgender Metallbleche, Streckmetall- oder Gewebelagen den gleichen Neigungswinkel, jedoch mit umgekehrtem Vorzeichen, aufweisen und dadurch Zellen ausbilden, die in vertikaler Richtung durch Engstellen zwischen den Knickkanten begrenzt werden.

Beispiele für Kreuzkanalpackungen sind Packungen der Typen Mellpack®, CY oder BX der Fa. Sulzer AG, CH-8404 Winterthur oder die Typen A3, BSH, B1 oder M der Fa. Monz GmbH, D-40723 Hilden.

In den Kreuzkanalpackungen bilden sich in vertikaler Richtung zwischen zwei jeweils aufeinander folgenden Metallblechen, Streckmetall- oder Gewebelagen, durch die geknickte Strukturierung derselben, Hohlräume, das heißt Zellen aus, die durch Engstellen zwischen den Knickkanten begrenzt werden.

Im Wirbelschichtreaktor, insbesondere in den Zellen bildenden Einbauten, sind Wärmeübertrager vorgesehen, in denen Wasser als Wärmeträger zirkuliert, das die Reaktionswärme der Hydrierung aufnimmt und hierbei verdampft. Die Wärmeübertrager können plattenförmig oder rohrförmig ausgebildet sein und im Wirbelschichtreaktor vertikal, horizontal oder geneigt angeordnet sein.

Verfahrensstufe IV

Das in Verfahrensstufe III erhaltene, Anilin enthaltende Produktgemisch wird in Verfahrensstufe IV bevorzugt destillativ aufgearbeitet, unter Erhalt eines Anilin enthaltenden Einsatzstromes, der in Verfahrensstufe V mit Formaldehyd in Gegenwart saurer Katalysatoren zu einem Methylendiphenylamin (MDA) enthaltenden Strom umgesetzt wird.

Verfahrensstufe V

Die Herstellung von MDA ist allgemein bekannt und erfolgt üblicherweise durch kontinuierliche oder diskontinuierliche Umsetzung von Anilin mit Formaldehyd in Gegenwart von sauren Katalysatoren. Bei dieser Umsetzung, deren Hauptprodukt das 4,4'-MDA ist, wird in geringem Ausmaß das unerwünschte Nebenprodukt N-Methyl-MDA gebildet. Dieses Nebenprodukt wirkt sich insbesondere bei der anschließenden Umsetzung des MDAs mit Phosgen zur Herstellung von Methylendi(phenylisocyanat), auch als MDI bezeichnet, negativ aus, da das N-Methyl-MDA die Vorläuferverbindung für chlorierte Nebenprodukte im MDI darstellt und möglichst geringe Gehalte an Chlor im MDI angestrebt werden.

Zur Verringerung von N-Methyl-MDA als Nebenprodukt bei der Herstellung von MDA sind verschiedene Verfahren bekannt:

Vorteilhaft wird die Verfahrensstufe V wie in EP-B 1 053 222 beschrieben durchgeführt, indem man in einem halbkontinuierlichen Verfahren Anilin und gegebenenfalls sauren Katalysator vorlegt, Formaldehyd und gegebenenfalls sauren Katalysator durch ein Mischorgan in einen Kreislauf, in dem Anilin, gegebenenfalls saurer Katalysator und gegebenenfalls bereits zugegebenes Formaldehyd kreisläufig bewegt werden, einspeist und nach Einspeisung von mindestens 50% der gesamten einzuspeisenden Formaldehydmenge die Reaktionsmischung auf eine Temperatur von größer 75°C temperiert. Diese Fahrweise erlaubt es, einen höheren Anteil an höheren MDA-Oligomeren zu erhalten als es mit einer kontinuierlichen Fahrweise bei hohen molaren Verhältnissen von Anilin zu Formaldehyd ohne Rückführung des MDA möglich ist. Dadurch ist eine Minimierung des Gehaltes an unerwünschten Nebenprodukten möglich.

Nach der bevorzugten Verfahrensweise für die Verfahrensstufe V wird ein Roh-MDI mit einem niedrigen Gehalt an hydrolisierbarem Chlor, von kleiner als 0,1%, insbesondere kleiner als 0,045%, sowie mit einer hellen Farbe, ausgedrückt durch eine Iodfarbzahl in einer Verdünnung von 1 : 5 in Monochlorbenzol von kleiner als 30, besonders bevorzugt von kleiner als 11, gewonnen.

Das Verfahrensprodukt der Verfahrensstufe V, das üblicherweise auch als Roh-MDA bezeichnet wird, d.h. ein Gemisch enthaltend Metyhlendi-phenylamin (MDA), beispielsweise 2,2'-, 2,4'-, und/oder 4,4'-MDA als monomeres MDA, und üblicherweise polymeres MDA, auch als Polymethylendi-(phenylamin) bezeichnet, enthält bevorzugt weniger als 0,09 Gew.-% N-Methyl MDA.

Verfahrensstufe VI

Der in Verfahrensstufe V erhaltene, MDA enthaltende Strom (Roh-MDA) wird in Verfahrensstufe VI bevorzugt destillativ aufgereinigt und in Verfahrensstufe VII einer Phosgenierung zu einem MDI enthaltenden Strom zugeführt.

Verfahrensstufe VII

Die Phosgenierung kann bevorzugt in üblichen, besonders bevorzugt in inerten Lösungsmitteln, z.B. chlorierten, aromatischen Kohlenwasserstoffen, beispielsweise Monochlorbenzol, Dichlorbenzole wie z.B. o-Dichlorbenzol, p-Dichlorbenzol, Trichlorbenzole, die entsprechende Toluole und Xylole, Chlorethylbenzol, Monochlordiphenyl, alpha- bzw. beta-Naphthylchlorid und Phthalsäuredialkylester, wie iso-Diethylphthalat, bevorzugt Toluol, Mono- und/oder Dichlorbenzol, in üblichen Reaktoren, beispielsweise Rührkesseln, Rührkesselkaskaden, Kolonnen und/oder Rohrreaktoren bei bekannten Temperaturen von z.B. 50 bis 150°C, bevorzugt 70 bis 120°C, besonders bevorzugt 70 bis 100°C und einem Druck von 0,5 bis 10 bar, bevorzugt 0,8 bis 5 bar, besonders bevorzugt 0,8 bis 1,5 bar in einer oder mehreren Stufen durchgeführt werden. Beispielsweise kann die Phosgenierung durch eine zweistufige Umsetzung in Gegenwart mindestens eines inerten organischen Lösungsmittels durchgeführt werden, wobei die erste Stufe der Phosgenierung in einem statischen Mischer und die zweite Stufe der Phosgenierung in einem Verweilzeitapparat durchgeführt wird und im Verweilzeitapparat die Massenverhältnisse von Phosgen zu Chlorwasserstoff gleichzeitig in der Flüssigphase 10-30: 1 und in der Gasphase 1-10: 1 betragen.

Als statischer Mischer für die erste Stufe der Phosgenierung kommen die bekannten Vorrichtungen, insbesondere Düsen, zur Anwendung.

Die Temperatur bei der ersten Stufe der Phosgenierung beträgt üblicherweise 50 bis 120°C, bevorzugt 60 bis 120°C, besonders bevorzugt 90 bis 120°C.

Als Verweilzeitapparat kommen die bekannten Apparate zur Anwendung, vorzugsweise Rührmaschinen, insbesondere Rührkesselkaskaden mit 2 bis 6 Rührkesseln, oder Kolonnen, insbesondere solche mit < 10 theoretischen Böden.

Bei der Verwendung von Rührmaschinen als Verweilzeitapparate werden, wie oben ausgeführt, insbesondere Rührkesselkaskaden mit mindestens 2, vorzugsweise 2 bis 6, besonders bevorzugt 2 bis 5 Rührkesseln, eingesetzt. Prinzipiell ist auch eine Kaskade mit mehr als 6 Rührkesseln einsetzbar, eine Vergrößerung der Zahl der Rührkessel über 6 steigert jedoch nur noch den apparativen Aufwand, ohne dass eine messbare Verbesserung des Endproduktes eintritt. Das Gemisch der ersten Stufe der Phosgenierung tritt üblicherweise mit einer Temperatur von 70 - 120°C, bevorzugt 85-105°C in die erste Rührmaschine ein. Die Temperaturen in den Rührmaschinen betragen bevorzugt, gemeinsam oder einzeln unterschiedlich, 75 - 120°C, besonders bevorzugt 80 - 110°C. Die Drücke in den Rührmaschinen betragen üblicherweise einzeln unterschiedlich oder gemeinsam 1,0-3,0 at Überdruck, bevorzugt 1,2-2,5 at Überdruck.

Das in Verfahrensstufe VII erhaltene, MDI enthaltende Produktgemisch, enthaltend Diphenylmethandiisocyanate (monomeres MDI) und Polyphenylen-polymethylen-polyisocyanate (polymeres MDI), hat üblicherweise einen Diphenylmethandiisocyanat-Isomerengehalt von 30 bis 90 Gew.-%, vorzugsweise von 30 bis 70 Gew.-%, einen NCO-Gehalt von 29 bis 33 Gew.-%, vorzugsweise 30 bis 32 Gew.-%, bezogen auf das Roh-MDI-Gewicht, und eine Viskosität, bestimmt gemäß DIN 51550 bei 25°C, von bevorzugt maximal 2500 mPa.s., vorzugsweise von 40 bis 2000 m Pa.s.

Das in Verfahrensstufe VII erhaltene, MDI enthaltende Produktgemisch (Roh-MDI) wird in Verfahrensstufe VIII bevorzugt destillativ zu Rein-MDI aufgearbeitet.

Verfahrensstufe VIII

Vorliegend wird als Rein-MDI ein Gemisch enthaltend mindestens 98,0 Gew.-% 4,4'-MDI und daneben maximal 2,0 Gew.-% 2,4'-MDI bezeichnet, wobei die Säurezahl, bestimmt nach ASTM D1638-74, maximal 10 ppm sein darf.

Bevorzugt kann der insbesondere destillativen Reinigung des in Verfahrensstufe VII erhaltenen, MDI enthaltenden Produktgemisches ein Strippprozess vorgeschaltet werden, bei dem Phosgen und gegebenenfalls Lösungsmittel aus dem Roh-MDI entfernt werden.

Bei einem solchen Strippprozess kann das Roh-MDI in einen oder mehrere Apparate mit großer innerer Oberfläche geleitet und auf dessen Oberfläche verteilt werden, so dass leichtflüchtige Komponenten entweichen können. Es kann sich bei der Apparatur beispielsweise und bevorzugt um einen Fallfilm- oder Dünnschichtverdampfer oder eine gepackte Kolonne geeigneter Auslegung handeln. Inertgase können als Strippmedium eingespeist und/oder Vakuum über die Apparatur angelegt werden. Die Temperaturen während dieses Stripprozesses betragen bevorzugt unter 210°C, besonders bevorzugt 50 bis 190°C.

Anschließend wird in Verfahrensstufe VIII destillativ Rein-MDI, beispielsweise bei Drücken von 2 bis 50 mbar, bevorzugt 2 bis 20 mbar, und Temperaturen von 150 bis 250°C, bevorzugt 180 bis 230°C, besonders bevorzugt 210 bis 230°C gewonnen.

Das Rein-MDI wird anschließend üblicherweise mit einem Antioxidans auf der Basis sterisch gehinderter Phenole und/oder mindestens einem Arylphosphit stabilisiert.

Erfindungsgemäß wird die stark exotherme Verfahrensstufe III, die katalytische Hydrierung von Nitrobenzol zu einem Anilin enthaltenden Produktgemisch in technisch einfacher, eleganter Weise genutzt, um Dampf auf zwei unterschiedlichen Druckniveaus zur Verfügung zu stellen, und damit die gesamte Anlage, umfassend sämtliche Verfahrensstufen I bis VIII, energetisch autark oder weitgehend autark zu betreiben.

Die komplexe Auslegung der Wirbelschichtreaktoren, die für die katalytische Hydrierung von Nitrobenzol eingesetzt werden, muss nach dem erfindungsgemäßen Verfahren lediglich einmal vorgenommen werden, da gefunden wurde, dass es möglich ist, in einfacher Weise Dampf auf zwei unterschiedlichen Druckstufen zur Verfügung zu stellen, indem ein erster Wirbelschichtreaktor mit Volllast betrieben wird, d.h. mit der Last, für die er ausgelegt wurde, und ein zweiter Wirbelschichtreaktor lediglich mit Teillast gefahren wird. Dadurch wird, bei gleicher Reaktionstemperatur, $T_R$, unverändertem Wärmedurchgangskoeffizienten k sowie gleicher Wärmeübertragungsfläche A eine geringere Wärmemenge Q erzeugt und somit ist die Temperatur des die Reaktionswärme abführenden Wärmeträgers $T_W$ und entsprechend der Dampfdruck desselben höher.

Die obigen Zusammenhänge können durch die Gleichung

$$Q = k \cdot A \cdot (T_R - T_W)$$

wiedergegeben werden, worin

Q   die durch die katalytische Hydrierung im Reaktor erzeugte Wärmemenge,

k   der Wärmedurchgangskoeffizient,

A   die Wärmeübertragungsfläche,

$T_R$   die Reaktionstemperatur und

$T_W$   die Temperatur des Wärmeübetragers

bedeuten.

Die Wärmemenge Q ist direkt proportional zur Produktionskapazität des Reaktors.

Der Wärmedurchgangskoeffizient k ist durch die Katalysatoreigenschaften bestimmt und variiert im Bereich von etwa 500 W/m²·K bis ca. 1000 W/m²·K. In der Regel steigt der k-Wert mit zunehmender Katalysatorstandzeit, insbesondere durch Verkokung des Katalysators und der damit verbundenen Erhöhung der Partikeldichte an.

Dies kann in einer bevorzugten Verfahrensvariante genutzt werden, indem die beiden Wirbelschichtreaktoren mit Katalysatoren befüllt werden, die unterschiedlich lange bereits im Einsatz waren, und zwar indem der mit frischerem Katalysator befüllte Wirbelschichtreaktor mit einer Last betrieben wird, die zur Erzeugung des Dampfes auf der niedrigeren Druckstufe führt, und der Wirbelschichtreaktor, der mit dem Katalysator befüllt ist, der bereits länger im Einsatz war, mit einer Last betrieben wird, die zur Erzeugung von Dampf auf einer höheren Druckstufe führt.

Erfindungsgemäß werden zwei Wirbelschichtreaktoren angesetzt, die baugleich sind, wobei unter "baugleich" nicht in allen Einzelheiten identische Apparate verstanden werden, sondern lediglich im Wesentlichen gleiche Apparate, insbesondere Apparate mit gleicher Wärmeübertragungsfläche A.

Die Reaktionstemperatur $T_R$ in den Wirbelschichtreaktoren wird insbesondere auf einen Wert im Bereich von ca. 280 bis 320°C, bevorzugt auf einen Wert im Bereich von ca. 290 bis 300°C, geregelt.

Bevorzugt wird das Wasser für die Abführung der Reaktionswärme aus dem Wirbelschichtreaktor über eine Dampftrommel einem im Innenraum des Wirbelschichtreaktors angeordneten Rohrbündelwärmetauscher zugeführt, und der durch die Aufnahme der Reaktionswärme durch Siedekühlung im Rohrbündelwärmetauscher entstehende Dampf nach außen über ein Regelventil abgeleitet, über das der Dampfdruck auf die erste bzw. zweite Druckstufe geregelt wird.

Bevorzugt wird die erste Druckstufe auf einen Wert im Bereich von etwa 16 bis 30 bar absolut geregelt.

Je nach Bedarf können anstelle eines einzigen ersten bzw. zweiten Wirbelschichtreaktors jeweils zwei oder mehrere Wirbelschichtreaktoren eingesetzt werden.

Es ist auch möglich, die Wirbelschichtreaktoren, die jeweils Dampf niedrigerer bzw. höherer Druckstufe liefern, während des laufenden Betriebs durch geeignete technische Maßnahmen flexibel zwischen den beiden Betriebsarten umzuschalten.

Darüber hinaus ist es auch möglich, mit der vorhandenen Anlage flexibel auf die Anilin-Nachfrage zu reagieren, indem die Anilin-Last des Wirbelschichtreaktors erhöht wird, der zuvor zur Erzeugung von Dampf auf höherer Druckstufe genutzt wurde, und stattdessen Dampf auf höherer Druckstufe von außen zugekauft wird.

Die Erfindung wird im Folgenden anhand eines Ausführungsbeispiels näher erläutert.

Auf Grundlage der vorstehend angegebenen Gleichung für die bei der katalytischen Hydrierung von Nitrobenzol erzeugte Wärmemenge,

$$Q = k \cdot A \cdot (T_R - T_W),$$

ergibt sich für einen Reaktor mit einer angestrebten Produktionskapazität von 120 kt/a sowie bei einer abzuführenden Reaktionswärme von 15,9 MW, einer Reaktortemperatur von 280°C, einem Wärmedurchgangskoeffizienten k von 550 W/m²·K und einem Dampftrommeldruck von 30 bar absolut eine benötigte Wärmetauscherfläche von 600 m².

Ein zweiter Wirbelschichtreaktor, mit gleicher Wärmeübertragungsfläche, von 600 m², wird mit entsprechend höherem Dampftrommeldruck, und zwar 42 bar absolut betrieben, um die Dampfversorgung in den Verfahrensstufen IV, VI und VIII zu gewährleisten. Aufgrund der kleineren Temperaturdifferenz zwischen Katalysatorbett und Wärmeübertrager kann eine geringere Wärmemenge abgeführt werden, von 10,5 MW, und entsprechend kann der zweite Wirbelschichtreaktor lediglich mit einer niedrigeren Kapazität, von 80 kt/a, d.h. mit einer Teillast, betrieben werden.

Somit wird durch Betrieb von zwei Wirbelschichtreaktoren mit gleicher Wärmeübetragungsfläche, von jeweils 600 m², wobei ein erster Reaktor mit Volllast und zweiter mit Teillast betrieben wird, Dampf auf zwei Druckstufen, und zwar von 30 bar absolut und Dampf 40 bar absolut, gewonnen.

**Patentansprüche**

1. Verfahren zur Herstellung und destillativen Aufarbeitung von Diphenylmethan-Diisocyanat (MDI), ausgehend von einem Benzol enthaltenden Einsatzstrom, wobei in

Verfahrensstufe I der Benzol enthaltende Einsatzstrom zu einem Nitrobenzol enthaltenden Produktgemisch nitriert, in

Verfahrensstufe II das Nitrobenzol enthaltende Produktgemisch bevorzugt destillativ aufgearbeitet wird, unter Erhalt eines Nitrobenzol enthaltenden Einsatzstromes, der in

Verfahrensstufe III eine katalytische Hydrierung zu einem Anilin enthaltenden Produktgemisch in einem Wirbelschichtreaktor, unter Abführung der Reaktionswärme durch Siedekühlung mit Wasser, wobei Dampf entsteht, zugeführt wird, wobei in

Verfahrensstufe IV das Anilin enthaltende Produktgemisch bevorzugt destillativ aufgearbeitet wird, unter Erhalt eines Anilin enthaltenden Einsatzstromes, der in

Verfahrensstufe V mit Formaldehyd in Gegenwart saurer Katalysatoren zu einem Methylendiphenylamin (MDA) enthaltenden Strom umgesetzt wird, der in
Verfahrensstufe VI bevorzugt destillativ aufgereinigt, und in
Verfahrensstufe VII einer Phosgenierung zu einem MDI enthaltenden Produktgemisch zugeführt wird, das in
Verfahrensstufe VIII bevorzugt destillativ zu Rein-MDI aufgearbeitet wird,
**dadurch gekennzeichnet, dass** in Verfahrensstufe III Dampf auf zwei unterschiedlichen Druckstufen erzeugt wird, der den Energiebedarf für die Verfahrensstufen IV, VI und VIII teilweise oder vollständig deckt, indem zwei baugleiche Wirbelschichtreaktoren eingesetzt werden, wobei

- ein erster Wirbelschichtreaktor mit einer Anilinlast betrieben wird, für die die Wirbelschichtreaktoren ausgelegt wurden und Dampf auf einer ersten, niedrigeren Druckstufe liefert, und
- ein zweiter Wirbelschichtreaktor mit einer gegenüber dem ersten Wirbelschichtreaktor soweit erniedrigter Last betrieben wird, dass der zweite Wirbelschichtreaktor Dampf auf der für die Verfahrensstufen IV, VI und VIII erforderliche höhere Druckstufe liefert.

2. Verfahren nach Anspruch 1, **dadurch gekennzeichnet, dass** das Wasser für die Abführung der Reaktionswärme aus dem Wirbelschichtreaktor über eine Dampftrommel einem im Innenraum des Wirbelschichtreaktors angeordneten Rohrbündelwärmetauscher zugeführt, und der durch die Aufnahme der Reaktionswärme durch Siedekühlung im Rohrbündelwärmetauscher entstehende Dampf nach außen über ein Regelventil abgeleitet wird, über das der Dampfdruck auf die erste bzw. auf die zweite Druckstufe geregelt wird.

3. Verfahren nach Anspruch 1 oder 2, **dadurch gekennzeichnet, dass** die erste Druckstufe auf einen Druck im Bereich von 16 bis 30 bar absolut und die zweite Druckstufe auf ≥ 40 bar absolut geregelt wird.

4. Verfahren nach einem der Ansprüche 1 bis 3, **dadurch gekennzeichnet, dass** die Reaktionstemperatur in den Wirbelschichtreaktoren auf einen Wert im Bereich von 280 bis 320°C geregelt wird.

5. Verfahren nach Anspruch 4, **dadurch gekennzeichnet, dass** die Reaktionstemperatur in den Wirbelschichtreaktoren auf einen Wert im Bereich von 290 bis 300°C geregelt wird.

6. Verfahren nach einem der Ansprüche 1 bis 5; wobei die beiden Wirbelschichtreaktoren mit Katalysatoren befüllt sind, die unterschiedlich lange bereits im Einsatz waren, **dadurch gekennzeichnet, dass** der mit frischerem Katalysator befüllte Wirbelschichtreaktor mit einer Last betrieben wird, die zur Erzeugung des Dampfes auf der niedrigeren Druckstufe führt, und der Wirbelschichtreaktor, der mit dem Katalysator befüllt ist, der bereits länger im Einsatz war, mit einer Last betrieben wird, die zur Erzeugung von Dampf auf der höheren Druckstufe führt.

**Claims**

1. A process for preparation and distillative workup of diphenylmethane diisocyanate (MDI), proceeding from a benzene-comprising feed stream, in which, in process stage I the benzene-comprising feed stream is nitrated to give a nitrobenzene-comprising product mixture, in
process stage II the nitrobenzene-comprising product mixture is worked up, preferably by distillation, to obtain a nitrobenzene-comprising feed stream which, in
process stage III, is supplied to a catalytic hydrogenation to give an aniline-comprising product mixture in a fluidized bed reactor with removal of the heat of reaction by evaporative cooling with water to form steam, in
process stage IV the aniline-comprising product mixture is worked up, preferably by distillation, to obtain an aniline-comprising feed stream which, in
process stage V, is reacted with formaldehyde in the presence of acidic catalysts to give a methylenediphenylamine (MDA)-comprising stream which, in
process stage VI, is purified, preferably by distillation, and, in
process stage VII, is supplied to a phosgenation to give an MDI-comprising product mixture which, in
process stage VIII, is worked up, preferably by distillation, to give pure MDI,
wherein, in process stage III, steam is raised at two different pressure levels, which partly or completely covers the energy demand for process stages IV, VI and VIII, by using two fluidized bed reactors of identical design, of which

- a first fluidized bed reactor is operated with an aniline load for which the fluidized bed reactors have been designed and provides steam at a first, lower pressure level, and
- a second fluidized bed reactor is operated with a load lowered with respect to the first fluidized bed reactor to such an extent that the second fluidized bed reactor affords steam at the higher pressure level required for process stages IV, VI and VIII.

2. The process according to claim 1, wherein the water for the removal of the heat of reaction from the fluidized bed reactor is supplied via a steam drum to a tube bundle heat exchanger arranged in the interior of the fluidized bed reactor, and the steam which arises from the absorption of the heat of reaction by evaporative cooling in the tube bundle heat exchanger is discharged via a regulating valve by which the steam pressure is regulated to the first or second pressure level.

3. The process according to claim 1 or 2, wherein the first pressure level is regulated to a pressure in the range from 16 to 30 bar absolute, and the second pressure level to $\geq$ 40 bar absolute.

4. The process according to any of claims 1 to 3, wherein the reaction temperature in the fluidized bed reactors is regulated to a value in the range from 280 to 320°C.

5. The process according to claim 4, wherein the reaction temperature in the fluidized bed reactors is regulated to a value in the range from 290 to 300°C.

6. The process according to any of claims 1 to 5, wherein the two fluidized bed reactors are filled with catalysts which have already been in use for different lengths of time, wherein the fluidized bed reactor filled with fresher catalyst is operated at a load which leads to raising of steam at the lower pressure level, and the fluidized bed reactor filled with the catalyst which has already been in use for a longer period is operated at a load which leads to raising of steam at the higher pressure level.

**Revendications**

1. Procédé de fabrication et de traitement par distillation de diisocyanate de diphénylméthane (MDI), à partir d'un courant d'entrée contenant du benzène, selon lequel
à l'étape de procédé I, le courant d'entrée contenant du benzène est nitré en un mélange de produits contenant du nitrobenzène,
à l'étape de procédé II, le mélange de produits contenant du nitrobenzène est de préférence traité par distillation pour obtenir un courant d'entrée contenant du nitrobenzène, qui
est introduit à l'étape de procédé III dans une hydrogénation catalytique pour former un mélange de produits contenant de l'aniline dans un réacteur à lit fluidisé, avec évacuation de la chaleur de réaction par refroidissement par ébullition avec de l'eau, de la vapeur se formant,
le mélange de produits contenant de l'aniline étant de préférence traité par distillation à l'étape de procédé IV pour obtenir un courant d'entrée contenant de l'aniline, qui
est mis en réaction à l'étape de procédé V avec du formaldéhyde en présence de catalyseurs acides pour former un courant contenant de la méthylène-diphénylamine (MDA), qui
est de préférence traité par distillation à l'étape de procédé VI, et
introduit à l'étape de procédé VII dans une phosgénation pour former un mélange de produits contenant du MDI, qui
est de préférence traité par distillation à l'étape de procédé VIII pour obtenir du MDI pur,
**caractérisé en ce qu'**à l'étape de procédé III, de la vapeur, qui couvre en partie ou en totalité la demande énergétique des étapes de procédé IV, VI et VIII, est formée à deux niveaux de pression différents en utilisant deux réacteurs à lit fluidisé de construction identique,

- un premier réacteur à lit fluidisé étant exploité avec une charge d'aniline, pour laquelle les réacteurs à lit fluidisé ont été conçus, et de la vapeur étant fournie à un premier niveau de pression inférieur, et
- un second réacteur à lit fluidisé étant exploité avec une charge réduite par rapport au premier réacteur à lit fluidisé de manière à ce que le second réacteur à lit fluidisé fournisse de la vapeur au niveau de pression plus élevé nécessaire pour les étapes de procédé IV, VI et VIII.

2. Procédé selon la revendication 1, **caractérisé en ce que** l'eau pour l'évacuation de la chaleur de réaction du réacteur à lit fluidisé est introduite par un tambour à vapeur dans un échangeur de chaleur à faisceau de tubes agencé dans l'espace intérieur du réacteur à lit fluidisé, et la vapeur formée par l'absorption de la chaleur de réaction par refroidissement par ébullition dans l'échangeur de chaleur à faisceau de tubes est évacuée vers l'extérieur par une soupape de régulation, grâce à laquelle la pression de la vapeur est régulée au premier ou au second niveau de pression.

3. Procédé selon la revendication 1 ou 2, **caractérisé en ce que** le premier niveau de pression est régulé à une pression dans la plage allant de 16 à 30 bar et le second niveau de pression à $\geq$ 40 bar.

4. Procédé selon l'une quelconque des revendications 1 à 3, **caractérisé en ce que** la température de réaction dans les réacteurs à lit fluidisé est régulée à une valeur dans la plage allant de 280 à 320 °C.

5. Procédé selon la revendication 4, **caractérisé en ce que** la température de réaction dans les réacteurs à lit fluidisé est régulée à une valeur dans la plage allant de 290 à 300 °C.

6. Procédé selon l'une quelconque des revendications 1 à 5, dans lequel les deux réacteurs à lit fluidisé sont remplis avec des catalyseurs, qui ont déjà été utilisés pendant des durées différentes, **caractérisé en ce que** le réacteur à lit fluidisé rempli avec le catalyseur le plus frais est exploité avec une charge qui conduit à la formation de la vapeur au niveau de pression plus bas, et le réacteur à lit fluidisé qui est rempli avec le catalyseur qui a déjà été utilisé le plus longtemps est exploité avec une charge qui conduit à la formation de la vapeur au niveau de pression plus élevé.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0771783 B
- WO 0164333 A
- DE 1114820
- DE 1133394

- DE 1114820 A
- DE 1133394 A
- WO 2008034770 A
- EP 1053222 B